# FASCICULE DE BREVET EUROPEEN

(11) **EP 1 655 277 B1**
(45) Date de publication et mention de la délivrance du brevet: **03.03.2010**
(21) Numéro de dépôt: 05292306.7
(22) Date de dépôt: 28.10.2005
(51) Int. Cl.: C07C 6/12

(54) **Procédé de transalkylation d'hydrocarbures alkylaromatiques mis en oeuvre dans deux zones réactionnelles**
Transalkylierung von alkylaromatischen Kohlenwasserstoffen in zwei Reaktionszonen
Transalkylation of alkylaromatic hydrocarbons in two reaction zones

(30) Priorité: 09.11.2004 FR 0411960
(43) Date de publication de la demande: 10.05.2006
(73) Titulaire: Institut Français du Pétrole, 92852 Rueil Malmaison Cedex (FR)
(72) Inventeur: Corma, Avelino, 46022 Valence (ES); Serra Alfaro, José Manuel, 46019 Valencia (ES); Guillon, Emmanuelle, 69390 Vernaison (FR)

(56) Documents cités:
- US-A- 5 030 787
- US-A- 5 942 651
- US-B1- 6 359 184

## Description

La présente invention se rapporte au domaine de la transformation d'hydrocarbures alkylaromatiques contenant au moins 9 atomes de carbone. Plus précisément, elle concerne un procédé de transalkylation d'hydrocarbures alkylaromatiques ayant au moins 9 atomes de carbone par molécule pour produire des xylènes.

La réaction de transalkylation consiste généralement à convertir une charge contenant du toluène et des hydrocarbures alkylaromatiques à au moins 9 atomes de carbone en vue de produire un mélange de xylènes (orthoxylène, métaxylène et paraxylène). Un autre composé aromatique à 8 atomes de carbone est également formé, l'éthylbenzène. Le paraxylène présente un réel intérêt industriel notamment pour l'industrie des textiles et il convient de le séparer des autres composés aromatiques à huit atomes de carbone. Ainsi, en sortie du réacteur de transalkylation, l'ensemble des produits formés est introduit dans une colonne de séparation de manière à séparer les composés à au moins six atomes de carbone des composés les plus légers. La fraction la plus lourde est soumise à plusieurs séparations de façon à extraire le benzène et le toluène puis l'effluent constitué de composés à au moins huit atomes de carbone est introduit dans une colonne de séparation permettant de récupérer en fond de colonne une fraction contenant essentiellement des composés à au moins neuf atomes de carbone et également une partie de l'orthoxylène et en tête de colonne une fraction formée de composés aromatiques à huit atomes de carbone (xylènes + éthylbenzène), cette fraction étant ensuite traitée dans des étapes d'isomérisation et de séparation du paraxylène. L'étape de séparation du paraxylène est d'autant plus facilitée que la proportion d'éthylbenzène dans ladite fraction formée de composés aromatiques à huit atomes de carbone est plus faible.

Les procédés de transalkylation d'hydrocarbures alkylaromatiques décrits dans l'art antérieur sont généralement des procédés en lit fixe. Les performances de tels procédés dépendent donc essentiellement des formulations catalytiques choisies ainsi que la nature de la charge et des conditions opératoires.

De nombreux catalyseurs de transalkylation ont déjà été décrits dans l'art antérieur, et sont à base de mordénite, de ZSM-5 ou bien aussi à base de zéolithe oméga.

Pour valoriser des charges aromatiques de plus en plus lourdes (ayant des groupements alkyles à plus d'un atome de carbone (éthyle, propyle...), des formulations catalytiques à base de catalyseurs composites ont été développées : par exemple, la demande de brevet FR-A-2,744,650 décrit l'utilisation d'un catalyseur composite pour la transalkylation d'hydrocarbures alkylaromatiques à base de zéolithes de type structural Mordénite et de type structural Mazzite. Le brevet US 5,942,651 décrit un système catalytique comprenant deux compositions catalytiques distinctes et séparées, l'une à base de zéolithe ayant un indice de contrainte compris entre 0,5 et 3 et contenant un métal noble et la seconde à base de zéolithe ayant un indice de contrainte compris entre 3 et 12 sans métal ajouté. Le brevet US 5,905,051 divulgue un système catalytique comprenant une première composition catalytique à base de zéolithe Béta promue par un métal et une seconde composition catalytique à base de zéolithe ZSM-5 sur laquelle est imprégnée un promoteur (S, P, Si). Le brevet US 5.030.787 divulgue un catalyseur comprenant une zéolithe ayant un indice de contrainte compris entre 1 et 3, en particulier une zéolithe MCM-22, ZSM-12 et beta. Le brevet US 6.359.184 divulgue une composition catalytique comprenant une zéolithe mordénite sous forme H, un support choisi parmi un oxyde inorganique et une argile, un composé métallique (Pt, Ni) déposé sur ledit support, ladite composition catalytique réalisant la transalkylation d'hydrocarbures alkylaromatiques à 9 atomes de carbone par molécule en toluène. Dans la plupart des procédés, la charge aromatique utilisée est constituée de benzène et/ou de toluène d'une part et d'hydrocarbures aromatiques lourds ayant au moins 9 atomes de carbone par molécule d'autre part. Le rendement en xylènes, produits par de tels procédés, mérite encore d'être amélioré. Aussi, la présente invention se propose de fournir un nouveau procédé de transalkylation conduisant à des performances catalytiques améliorées par rapport à celles obtenues par les procédés de l'art antérieur, notamment en terme de rendement en xylènes.

### Résumé et intérêt de l'invention :

L'objet de la présente invention est un procédé de production de xylènes par transalkylation d'une charge d'hydrocarbures alkylaromatiques ayant au moins 9 atomes de carbone par molécule comprenant :
a) l'introduction de ladite charge d'hydrocarbures alkylaromatiques à l'entrée d'une première zone réactionnelle où elle est mise en contact avec au moins un premier catalyseur zéolithique, les hydrocarbures alkylaromatiques ayant au moins 9 atomes de carbone par molécule représentant au moins 95% en volume de ladite charge,
b) l'introduction d'au moins une partie de l'effluent issu de l'étape a) et d'une charge contenant uniquement du toluène pur à l'entrée d'une deuxième zone réactionnelle contenant au moins un deuxième catalyseur zéolithique et
c) la séparation d'au moins une partie de l'effluent issu de l'étape b).

Le procédé selon la présente invention se révèle très efficace pour la transalkylation d'hydrocarbures alkylaromatiques alkylaromatiques AC₉⁺ (c'est-à-dire à au moins 9 atomes de carbone par molécule), car il permet de traiter des charges contenant une grande quantité d'aromatiques lourds AC₉⁺, ces aromatiques lourds pouvant contenir une grande proportion d'AC₁₀⁺. Ainsi, des charges AC₉⁺ contenant au moins 5 % et jusqu'à 25 % poids, et même davantage d'AC₁₀⁺ peuvent être valorisées. A titre d'exemples, on peut citer de manière non exhaustive, les diméthyléthylbenzènes, les diéthylbenzènes, les propyléthylbenzènes... Il a été découvert de manière surprenante par la Demanderesse que l'introduction d'une charge AC9+, dépourvue de benzène et/ou de toluène ou présent(s) à l'état de traces, dans une première zone réactionnelle et l'introduction de toluène pur dans une deuxième zone réactionnelle, placée en aval de ladite première zone conduit à une amélioration du rendement en xylènes par rapport à celui obtenu par les procédés antérieurs dans lesquels la charge AC9+ et le toluène sont introduits simultanément en mélange dans une même charge. Par ailleurs, il a également été découvert de manière très surprenante et avantageuse que, en comparaison avec les procédés connus, le procédé selon l'invention permet de réduire la quantité de catalyseur nécessaire pour réaliser la réaction de transalkylation tout en maintenant des performances catalytiques, notamment en terme de rendement en xylènes, satisfaisantes.

### Description de l'invention :

L'objet de la présente invention est un procédé de production de xylènes par transalkylation d'une charge d'hydrocarbures alkylaromatiques ayant au moins 9 atomes de carbone par molécule comprenant :
a) l'introduction de ladite charge d'hydrocarbures alkylaromatiques à l'entrée d'une première zone réactionnelle où elle est mise en contact avec au moins un premier catalyseur zéolithique, les hydrocarbures alkylaromatiques ayant au moins 9 atomes de carbone par molécule représentant au moins 95% en volume de ladite charge,
b) l'introduction d'au moins une partie de l'effluent issu de l'étape a) et d'une charge contenant uniquement du toluène pur à l'entrée d'une deuxième zone réactionnelle contenant au moins un deuxième catalyseur zéolithique et
c) la séparation d'au moins une partie de l'effluent issu de l'étape b).

L'installation permettant de réaliser le procédé de transalkylation selon l'invention comprend au moins deux zones réactionnelles distinctes, contenant chacune au moins un catalyseur zéolithique. Chacune desdites zones réactionnelles peut être comprise dans un seul réacteur catalytique, la première zone réactionnelle à l'entrée de laquelle est introduite ladite charge d'hydrocarbures alkylaromatiques étant située en amont de la deuxième zone réactionnelle à l'entrée de laquelle sont introduites au moins une partie de l'effluent sortant de ladite première zone réactionnelle et une charge contenant uniquement du toluène pur. L'introduction de la charge contenant du toluène pur peut, par exemple, se faire par injection latérale dans le réacteur dans une zone située entre la première et la deuxième zones réactionnelles. Chacune desdites zones réactionnelles peut également se trouver dans un réacteur catalytique distinct : la première zone réactionnelle à l'entrée de laquelle est introduite ladite charge d'hydrocarbures alkylaromatiques se trouve alors dans un premier réacteur catalytique et la deuxième zone réactionnelle à l'entrée de laquelle sont introduites au moins une partie de l'effluent sortant de ladite première zone réactionnelle et une charge contenant uniquement du toluène pur se trouve dans un deuxième réacteur catalytique, placé en aval dudit premier réacteur et en série. L'introduction de la charge contenant uniquement du toluène pur peut, par exemple, se faire par injection latérale entre le premier et le deuxième réacteurs.

La charge d'hydrocarbures alkylaromatiques introduite à l'entrée de la première zone réactionnelle contient des composés aromatiques ayant au moins 9 atomes de carbone par molécule (charge AC9+). Cette charge provient avantageusement d'un procédé de production de composés aromatiques fonctionnant en boucle et générant des hydrocarbures alkylaromatiques ayant au moins 9 atomes de carbone par molécule en tant que sous-produits. Elle contient notamment des hydrocarbures alkylaromatiques à 9 atomes de carbone tels que l'éthyltoluène et les triméthylbenzènes et des hydrocarbures alkylaromatiques à au moins 10 atomes de carbone. Elle contient essentiellement des hydrocarbures alkylaromatiques ayant au moins 9 atomes de carbone, représentant au moins 95% en volume de la charge, mais peut aussi contenir du benzène et/ou du toluène à l'état de traces (quelques ppm). De manière très préférée, elle ne contient que des hydrocarbures alkylaromatiques ayant au moins 9 atomes de carbone par molécule.

La charge contenant du toluène introduite à l'entrée de la deuxième zone réactionnelle conformément à l'étape b) du procédé selon l'invention est constituée uniquement de toluène pur. De manière très préférée, elle est constituée uniquement de toluène pur provenant en général de la distillation d'une coupe aromatique provenant du reformage catalytique et de la pyrolyse (vapocraquage) des naphtas.

Conformément à l'invention, la quantité de charge d'hydrocarbures alkylaromatiques utilisée pour l'étape a) et la quantité de charge contenant du toluène pur utilisée pour l'étape b) sont telles que le mélange de ces deux charges, sans transformation préalable, contiendrait de 1 à 99 % de AC9+ par rapport au mélange total.

Pour la mise en oeuvre du procédé selon l'invention, il est introduit un premier flux d'hydrogène à l'entrée de ladite première zone réactionnelle et un deuxième flux d'hydrogène à l'entrée de ladite deuxième zone réactionnelle.

Conformément à l'étape b) du procédé selon l'invention, la charge contenant uniquement du toluène pur est introduite à l'entrée de la deuxième zone réactionnelle avec au moins une partie de l'effluent issu de l'étape a). Avantageusement, ledit effluent est mélangé au moins en partie à ladite constituée uniquement de toluène pur, en amont de la deuxième zone réactionnelle afin de constituer la charge introduite à l'entrée de ladite deuxième zone réactionnelle. Ledit effluent issu de l'étape a) du procédé selon l'invention contient généralement des hydrocarbures alkylaromatiques à au moins 9 atomes de carbone par molécule non convertis ainsi que du benzène, du toluène et des xylènes produits au cours de l'étape a). De préférence, ledit effluent issu de l'étape a) est introduit en intégralité à l'entrée de la deuxième zone réactionnelle.

Les conditions opératoires (température, pression, débit) mises en oeuvre dans les étapes a) et b) du procédé selon l'invention sont celles connues de l'Homme du métier. Elles peuvent être identiques ou différentes dans la première et la deuxième zones réactionnelles.

Dans chacune des zones réactionnelles, la température est comprise entre 250 et 650°C et de préférence entre 350 et 550°C, la pression est comprise entre 1 et 6 MPa et de préférence entre 2 et 4,5 MPa, la vitesse spatiale d'alimentation par zone réactionnelle, exprimée en kilogramme de charge introduite par kilogramme de catalyseur et par heure, est comprise entre 0,05 et 20 h⁻¹ et de préférence entre 0,5 et 10 h⁻¹ et un rapport molaire hydrogène sur hydrocarbures aromatiques (H₂/HC aromatiques) compris entre 1 et 30 et de préférence entre 3 et 12 mol/mol. On définit la vitesse spatiale d'alimentation dans la première zone réactionnelle WHSV1 = (débit massique de la charge d'hydrocarbures alkylaromatiques à au moins 9 atomes de carbone) / (masse du premier catalyseur zéolithique). On définit la vitesse spatiale d'alimentation dans la deuxième zone réactionnelle WHSV2 = (débit massique de la charge contenant du toluène pur + débit massique de l'effluent issu de la première zone réactionnelle) / (masse du deuxième catalyseur zéolithique).

On définit aussi une vitesse spatiale d'alimentation globale (WHSV) sur l'ensemble de la première et de la deuxième zones réactionnelles du procédé selon l'invention comprise entre 0,05 et 20 h⁻¹. La vitesse spatiale d'alimentation globale WHSV est déterminée par la formule :
WHSV = (débit massique de la charge contenant du toluène pur + débit massique de la charge AC9+) / (masse du premier catalyseur zéolithique + masse du deuxième catalyseur zéolithique). On définit aussi un rapport molaire global H₂/HC aromatiques sur l'ensemble de la première et de la deuxième zones réactionnelles du procédé selon l'invention compris entre 1 et 30 et de préférence entre 3 et 12. Ce rapport molaire global H₂/HC aromatiques est déterminé par la formule : H₂/HC aromatiques = (nombre de moles d'H₂ introduites dans la première zone réactionnelle et la deuxième zone réactionnelle)/(nombre de moles d'hydrocarbures aromatiques introduites dans la première zone réactionnelle et la deuxième zone réactionnelle).

La réaction de transalkylation mise en oeuvre dans la première et la deuxième zones réactionnelles conformément aux étapes a) et b) du procédé selon l'invention est suivie d'au moins une étape de séparation c) d'au moins une partie, de préférence de la totalité, de l'effluent issu de l'étape b) afin de récupérer les réactifs en excès d'une part, c'est-à-dire les hydrocarbures alkylaromatiques à au moins 9 atomes de carbone, le toluène, et les xylènes d'autre part. Plus précisément, à la sortie de la deuxième zone réactionnelle utilisée pour la mise en oeuvre de l'étape b) du procédé selon l'invention, on fractionne en général le produit obtenu de manière à recueillir séparément un premier effluent renfermant le toluène, non converti, un deuxième effluent renfermant les composés aromatiques à 8 atomes de carbone notamment les xylènes, un troisième effluent renfermant les hydrocarbures alkylaromatiques ayant au moins 9 atomes de carbone et non convertis et un quatrième effluent renfermant des paraffines légères ayant de 1 à 6 atomes de carbone.

Conformément aux étapes a) et b) du procédé selon l'invention, la première zone réactionnelle contient au moins un premier catalyseur zéolithique et la deuxième zone réactionnelle contient au moins un deuxième catalyseur zéolithique. De tels catalyseurs sont connus de l'Homme du métier pour réaliser des transformations d'hydrocarbures à leur surface.

Ledit premier catalyseur zéolithique et ledit deuxième catalyseur zéolithique présents respectivement dans ladite première zone réactionnelle et ladite deuxième zone réactionnelle comprennent avantageusement au moins une zéolithe de structure cristalline ayant par exemple une structure telle que définie dans la classification "Atlas of Zeolite Structure Types" (W. M Meier, D. H. Olson and Ch. Baerlocher, 5th revised edition, 2001, Elsevier) auquel se réfère également la présente demande. Les zéolithes y sont classées selon la taille de leurs ouvertures de pores ou canaux. Chaque zéolithe présente dans ledit premier catalyseur zéolithique et ledit deuxième catalyseur zéolithique comporte au moins un élément X choisi parmi le silicium et le germanium et au moins un élément T choisi parmi l'aluminium, le fer, le gallium, le bore, le titane, le vanadium, le zirconium, le molybdène, l'arsenic, l'antimoine, le chrome et le manganèse.

De préférence, ledit premier catalyseur zéolithique et ledit deuxième catalyseur zéolithique comprennent au moins une zéolithe choisie dans le groupe constituée par les zéolithes de type structural MOR, BEA, MFI, EUO, FAU, BOG, TON et NES. Parmi les zéolithes de type structural MOR, la zéolithe mordénite est préférée. Parmi les zéolithes de type structural BEA, la zéolithe beta est préférée. Parmi les zéolithes de type structural MFI, la zéolithe ZSM-5 est préférée. Parmi les zéolithes de type structural EUO, la zéolithe EU-1 est préférée. Parmi les zéolithes de type structural FAU, la zéolithe Y et la zéolithe Y échangée avec des terres rares (REY) sont préférées. Parmi les zéolithes de type structural BOG, la zéolithe boggsite est préférée. Parmi les zéolithes de type structural TON, la zéolithe ZSM-22 est préférée. Parmi les zéolithes de type structural NES, la zéolithe NU-87 est préférée. On peut aussi avantageusement choisir comme zéolithe comprise dans ledit premier et/ou ledit deuxième catalyseur zéolithique une zéolithe IM-5 (FR-A-2.754.809 ou US 6.136.290). Avantageusement, la zéolithe présente dans ledit premier et ledit deuxième catalyseurs zéolithiques sont sous forme acide.

Les zéolithes présentes dans ledit premier catalyseur zéolithique et ledit deuxième catalyseur zéolithique peuvent être calcinées et échangées par au moins un traitement par une solution d'au moins un sel d'ammonium de manière à obtenir la forme ammonium des zéolithes qui une fois calcinées conduit à la forme hydrogène desdites zéolithes. Lesdites zéolithes sont au moins en partie, de préférence pratiquement totalement, sous forme acide, c'est-à-dire sous forme hydrogène (H⁺). Le rapport atomique Na/T est généralement inférieur à 10% et de préférence inférieur à 5% et de manière encore plus préférée inférieur à 1 %.

Selon un mode de réalisation de l'invention, ledit premier catalyseur zéolithique et ledit deuxième catalyseur zéolithique comprennent une même et une seule zéolithe, c'est-à-dire une zéolithe de même type structural et ayant la même composition chimique de leur charpente cristalline (même rapport X/T où X et T ont les mêmes définitions que ci-dessus) dans ledit premier catalyseur zéolithique et ledit deuxième catalyseur zéolithique. De préférence, il s'agit d'une zéolithe de type structural MFI, en particulier la zéolithe ZSM-5.

Selon un autre mode de réalisation de l'invention, ledit premier catalyseur zéolithique diffère dudit deuxième catalyseur zéolithique. Par exemple, ledit premier catalyseur zéolithique peut comprendre une zéolithe qui présente un type structural et/ou une composition chimique de sa charpente cristalline (rapport X/T) différente de celle comprise dans ledit deuxième catalyseur zéolithique. De manière très préférée, le premier catalyseur zéolithique comprend au moins une zéolithe présentant des canaux dont l'ouverture est définie par un anneau à 10 atomes d'oxygène (ouverture à 10MR) et le deuxième catalyseur zéolithique comprend au moins une zéolithe présentant des canaux dont l'ouverture est définie par un anneau à 10 atomes d'oxygène (ouverture à 10MR) et au moins une zéolithe présentant au moins des canaux ou des poches latérales dont l'ouverture est définie par un anneau à 12 atomes d'oxygène (ouverture à au moins 12MR). Selon l'invention, les canaux des zéolithes ayant une ouverture à 10 MR appelées dans la suite de la description par zéolithes 10 MR sont des canaux principaux qui débouchent directement sur l'extérieur desdites zéolithes. La zéolithe ayant une ouverture à au moins 12 MR appelée dans la suite de la description par zéolithe à au moins 12 MR présente au moins soit des canaux principaux 12 MR débouchant directement sur l'extérieur de ladite zéolithe soit des canaux secondaires 12 MR accessibles uniquement par des canaux principaux ayant une ouverture autre que 12 MR soit encore des poches latérales ("side pockets") dont l'ouverture est définie par un anneau à 12 atomes d'oxygène. Les zéolithes 10MR présentent dans lesdits premier et deuxième catalyseurs zéolithiques et la zéolithe à au moins 12MR présente dans ledit deuxième catalyseur zéolithique comprennent au moins un élément X et au moins un élément T, où X et T ont les mêmes définitions que celles données plus haut dans la description de la présente invention. De manière avantageuse, X est le silicium et T est l'aluminium. Elles sont, de préférence, pratiquement totalement, sous forme acide.

La zéolithe 10 MR présente dans le premier catalyseur zéolithique et le deuxième catalyseur zéolithique employés pour la mise en oeuvre des étapes a) et b) du procédé selon l'invention est caractérisée par un rapport Si/Al compris entre 2 et 250, de préférence compris entre 5 et 150 et de manière très préférée compris entre 10 et 80. La teneur en sodium est inférieure à 0,1 % poids, de préférence inférieure à 0,05 % poids par rapport au poids total de zéolithe sèche. Toutes les zéolithes possédant des canaux dont l'ouverture est définie par un anneau à 10 atomes d'oxygène (10 MR) et connues dans l'art antérieur conviennent pour la mise en oeuvre desdits premier et deuxième catalyseurs zéolithiques. Les zéolithes 10 MR préférées sont choisies parmi les zéolithes ZSM-5, IM-5 et ZSM-22. La zéolithe IM-5 est connue de l'Homme du métier comme pouvant être assimilée à une zéolithe présentant des canaux dont l'ouverture est définie par un anneau à 10 atomes d'oxygène (J.Catal 189(2000)382-394, A.Corma et al). Ces zéolithes 10 MR décrites ci-dessus et leur mode de préparation sont bien connus de l'Homme du métier.

La zéolithe à au moins 12MR présente dans le deuxième catalyseur zéolithique employé pour la mise en oeuvre de l'étape b) du procédé selon l'invention est caractérisée par un rapport Si/Al compris entre 2 et 250, de préférence compris entre 5 et 150 et de manière très préférée compris entre 10 et 80. La teneur en sodium est inférieure à 0,1 % poids, de préférence inférieure à 0,05% poids par rapport au poids total de zéolithe sèche. Toutes les zéolithes possédant au moins des canaux (principaux ou secondaires) ou des poches latérales dont l'ouverture est définie par un anneau à 12 atomes d'oxygène (12 MR) et connues dans l'art antérieur conviennent pour la mise en oeuvre dudit deuxième catalyseur zéolithique. Les zéolithes à au moins 12 MR, préférées dans le cadre de la présente invention, sont choisies parmi les zéolithes Béta, Y, mordénite, NU-87, ITQ-23, EU-1 et la boggsite. La zéolithe NU-87, de type structural NES possède des canaux principaux à 10MR et également des canaux secondaires à 12MR accessibles par les canaux à 10MR, telle que décrit dans le livre "Synthesis of microporous materials", Vol1, Ed M.L.Occelli and H.E.Robson, chap 24 (Casci J.L et al). La boggsite possède des canaux principaux à 10MR et 12 MR. La zéolithe EU-1 possède des canaux principaux à 10 MR et des poches latérales ("side pockets") à 12 MR. Ces zéolithes et leur mode de préparation sont bien connus de l'Homme du métier. La zéolithe ITQ-23 est décrite dans "An study of cyclohexylpyrrolidine-derived quaternary organic cations as structure directing agents for synthesis of zeolites", A. Corma, 1. Giménez, S. Leiva, F. Rey, M.J. Sabater, G. Sastre, S. Valencia, "Abstracts of 14th Int. Zeolite Conference", (Eds. E. van Stee, L. H. Callanan and M. Claeys), 25-30 April 2004, Cape Town, South Africa.

S'agissant dudit deuxième catalyseur zéolithique utilisé dans l'étape b) du procédé selon l'invention et dans le cas où il comporte au moins une zéolithe 10 MR et au moins une zéolithe à au moins 12 MR, la répartition entre les deux zéolithes (zéolithe 10 MR et zéolithe à au moins 12 MR) est telle que la teneur en zéolithe(s) choisie(s) dans le groupe formé par les zéolithes de type 10MR peut varier de 1% à 99%, de préférence de 5 à 95%, et de manière encore plus préférée peut varier entre 10 et 90% en pourcentages relatifs de l'ensemble des zéolithes introduites dans le deuxième catalyseur. De même, la teneur en zéolithe à au moins 12MR varie de 1% à 99%, de préférence de 5 à 95% et de manière encore plus préférée varie entre 10 et 90%, en pourcentages relatifs, de l'ensemble des zéolithes introduites dans le deuxième catalyseur.

Les rapports Si/Al des zéolithes 10MR et au moins 12MR décrites ci-dessus sont ceux obtenus à l'issue de la synthèse desdites zéolithes ou bien obtenus après des traitements de désalumination post-synthèse bien connus de l'homme du métier, tels que et à titre non exhaustif les traitements hydrothermiques suivis ou non d'attaques acides ou bien encore les attaques acides directes par des solutions d'acides minéraux ou organiques.

Le rapport Si/AI des zéolithes 10 MR et au moins 12 MR entrant dans la composition desdits premier et deuxième catalyseurs zéolithiques ainsi que la composition chimique des échantillons sont déterminés par fluorescence X et absorption atomique.

On peut également envisager d'utiliser comme premier catalyseur zéolithique, un catalyseur comprenant une zéolithe 10 MR et une zéolithe à au moins 12MR, et comme deuxième catalyseur zéolithique, un catalyseur comprenant une zéolithe 10 MR.

Le premier catalyseur zéolithique et/ou le deuxième catalyseur zéolithique employés pour la mise en oeuvre des étapes a) et b) du procédé selon l'invention comprend / comprennent avantageusement au moins un métal. Ledit métal est choisi dans le groupe constitué par les métaux des groupes IIIA, VIB, VIIB et VIII, de préférence à une teneur comprise entre 0,01 et 5 % poids par rapport au poids total du catalyseur zéolithique. Parmi les métaux du groupe IIIA, le gallium est préféré. Parmi les métaux du groupe VIB, le molybdène est préféré. Parmi les métaux du groupe VIIB, le rhénium est préféré. Parmi les métaux du groupe VIII, le nickel est préféré. De manière très avantageuse, le métal préféré parmi les métaux des groupes IIIA, VIB, VIIB et VIII est le rhénium. Ledit métal peut être présent soit dans la composition du premier catalyseur zéolithique soit dans la composition du deuxième catalyseur zéolithique soit encore dans la composition du premier et du deuxième catalyseurs zéolithiques. Il est très avantageux d'utiliser dans l'étape a) du procédé selon l'invention un premier catalyseur zéolithique comprenant au moins un premier métal choisi dans la liste citée ci-dessus et dans l'étape b) du procédé selon l'invention un deuxième catalyseur zéolithique comprenant au moins un deuxième métal choisi dans la liste citée ci-dessus et différent dudit premier métal. Ledit premier catalyseur zéolithique et/ou ledit deuxième catalyseur zéolithique employés pour la mise en oeuvre des étapes a) et b) du procédé selon l'invention comprend /comprennent éventuellement en outre au moins un métal du groupe IVA, de préférence l'étain, à une teneur comprise avantageusement entre 0,01 et 5 % et de préférence entre 0,5 et 3 % poids par rapport au poids total du catalyseur.

Les catalyseurs zéolithiques utilisés pour la mise en oeuvre du procédé selon l'invention sont mis en forme. La mise en forme peut être réalisée avec d'autres matrices que l'alumine, telles que par exemple la magnésie, les silice-alumines amorphes, les argiles naturelles (kaolin, bentonite, sepiolite, attapulgite), la silice, l'oxyde de titane, l'oxyde de bore, la zircone, les phosphates d'aluminium, les phosphates de titane, les phosphates de zirconium, le charbon et leurs mélanges. On préfère utiliser des matrices contenant de l'alumine, sous toutes ces formes connues de l'Homme du métier, et de manière encore plus préférée l'alumine gamma. On peut aussi avantageusement utiliser des mélanges d'alumine et de silice, des mélanges d'alumine et de silice-alumine. D'autres techniques que l'extrusion, telles que le pastillage ou la dragéification, peuvent être utilisées pour la mise en forme.

Les catalyseurs zéolithiques utilisés pour la mise en oeuvre du procédé selon l'invention sont mis en forme sous la forme de grains de différentes formes et dimensions. Ils sont utilisés en général sous la forme d'extrudés cylindriques ou polylobés tels que bilobés, trilobés, polylobés de forme droite ou torsadée, mais peuvent éventuellement être fabriqués et employés sous la forme de poudre concassées, de tablettes, d'anneaux, de billes, de roues.

Le/les métal/métaux présent(s) dans la composition du premier et/ou du deuxième catalyseur(s) zéolithique(s) peu(ven)t être déposé(s) sur la zéolithe ou sur la matrice du catalyseur zéolithique concerné. Pour le dépôt de métal sur la zéolithe, on utilise en général la technique d'échange cationique, la technique d'imprégnation à sec ou de coprécipitation. Pour le dépôt de métal non plus directement sur la zéolithe mais sur la matrice minérale poreuse (avant ou après la mise en forme), on utilise en général la technique d'échange anionique. Dans le cas où au moins l'un des catalyseurs zéolithiques contient plusieurs métaux, ces derniers peuvent être introduits soit tous de la même façon soit par des techniques différentes, avant ou après mise en forme et dans n'importe quel ordre. Dans le cas où la technique utilisée est celle de l'échange ionique (anionique ou cationique), plusieurs échanges successifs peuvent être nécessaires pour introduire les quantités requises de métaux.

Les sources des métaux du groupe VIII qui peuvent être utilisées sont bien connues de l'Homme du métier. Par exemple, on utilisera les nitrates, les sulfates, les phosphates, les halogénures par exemples chlorures, bromures et fluorures, les carboxylates par exemple acétates et carbonates. Dans le cas du nickel, on peut utiliser préférentiellement du nitrate de nickel Ni(NO₃)₂. Les sources des métaux du groupe VIIB qui peuvent être utilisées sont également bien connues de l'Homme du métier. Dans le cas du rhénium, on utilise habituellement un complexe de perrhénate d'ammonium (NH₄)ReO₄ ou l'acide perrhénique. Les sources des métaux du groupe IIIA qui peuvent être utilisées sont également bien connues de l'Homme du métier. Dans le cas du gallium, le nitrate de gallium Ga(NO₃)₃ est préféré. Les sources des métaux du groupe VIB qui peuvent être utilisées sont également bien connues de l'Homme du métier. Dans le cas du molybdène, on peut utiliser les acides molybdiques et leurs sels en particulier les sels d'ammonium tels que le molybdate d'ammonium, l'heptamolybdate d'ammonium ainsi que l'acide phosphomolybdique. De préférence, on utilise l'heptamolybdate d'ammonium (NH₄)₆Mo₇O₂₄. Le dépôt du métal ou des métaux des groupes IIIA, VIB, VIIB et VIII et éventuellement du groupe IVA est suivi en général d'une calcination sous air ou oxygène, usuellement entre 300 et 600°C durant 0,5 à 10 heures, de préférence entre 350°C et 550°C durant 1 à 4 heures. On peut procéder ensuite à une réduction sous hydrogène, généralement à une température comprise entre 300 et 600°C pendant 1 à 10 heures, de préférence on opérera entre 350° et 550°C pendant 2 à 5 heures.

Selon le mode de réalisation de l'invention décrit ci-dessus consistant à utiliser dans l'étape a) du procédé selon l'invention au moins un premier catalyseur zéolithique comprenant au moins une zéolithe 10MR et dans l'étape b) du procédé selon l'invention au moins un deuxième catalyseur zéolithique comprenant au moins une zéolithe 10MR et au moins une zéolithe à au moins 12MR, on donne ci-après trois modes de préparation du deuxième catalyseur zéolithique. Selon une première variante de préparation dudit deuxième catalyseur zéolithique, préalablement à leur mise en forme, au moins une des zéolithes précédemment décrites et comprise dans ledit deuxième catalyseur est soumise au dépôt d'au moins un métal choisi dans le groupe constitué par les métaux des groupes IIIA, VIB, VIIB et VIII. De préférence, au moins la zéolithe 10 MR est soumise au dépôt d'au moins un métal choisi dans le groupe constitué par les métaux des groupes IIIA, VIB, VIIB et VIII. Il est aussi possible que la zéolithe 10 MR soit soumise au dépôt d'un métal choisi dans le groupe constitué par les métaux des groupes IIIA, VIB, VIIB et VIII et que la zéolithe à au moins 12 MR soit soumise au dépôt d'un autre métal choisi dans le groupe constitué par les métaux des groupes IIIA, VIB, VIIB et VIII. Avantageusement, lorsque ledit deuxième catalyseur zéolithique comporte au moins un métal choisi dans le groupe constitué par les métaux des groupes IIIA, VIB, VIIB et VIII et au moins un métal choisi dans le groupe IVA, la zéolithe 10 MR est soumise au dépôt du métal choisi dans le groupe constitué par les métaux des groupes IIIA, VIB, VIIB et VIII et la zéolithe à au moins 12 MR est soumise au dépôt du métal choisi dans le groupe constitué par les métaux du groupe IVA. Les zéolithes ainsi chargées en métaux sont mélangées. Le mélange de ces zéolithes qui sont alors à l'état de poudre est réalisé par toutes les techniques de mélange de poudres connues de l'homme du métier. Une fois le mélange des poudres de zéolithes, chargées en métaux, réalisé, le mélange est mis en forme par toute technique connue de l'homme de l'art. Il peut en particulier être mélangé à une matrice minérale poreuse, généralement amorphe, par exemple à une poudre humide de gel d'alumine. Le mélange est ensuite mis en forme, par exemple par extrusion au travers d'une filière, par pastillage ou par dragéification. La matrice minérale poreuse est du même type que celle décrite plus haut dans la présente description. Après l'étape de mise en forme, le produit obtenu est soumis à une étape de séchage puis à une étape de calcination.

Selon une deuxième variante de préparation dudit deuxième catalyseur zéolithique, au moins un métal choisi dans le groupe constitué par les métaux des groupes IIIA, VIB, VIIB et VIII et éventuellement au moins un métal choisi dans le groupe IVA est(sont) déposé(s) sur le support après la mise en forme des zéolithes 10MR et à au moins 12MR, exemptes de métaux, par tout procédé connu de l'homme du métier et permettant le dépôt du métal sur zéolithes. On désigne par le terme "support", le mélange de zéolithes (exemptes de métaux) avec au moins une matrice minérale poreuse après mise en forme, séchage et calcination. Le support dudit deuxième catalyseur zéolithique utilisé dans l'étape b) du procédé selon l'invention renferme généralement les teneurs suivantes en matrice et zéolithes :
- 5 à 95% poids, de préférence 10 à 90% poids, de manière plus préférée de 15 à 85% poids et de manière très préférée de 20 à 80% poids de zéolithes telles que au moins une zéolithe soit choisie parmi les zéolithes 10MR et au moins une zéolithe soit choisie parmi les zéolithes à au moins 12MR,
- 5 à 95%, de préférence de 10 à 90%, de manière plus préférée de 15 à 85% et de manière très préférée de 20 à 80% poids d'au moins une matrice minérale poreuse amorphe ou mal cristallisée de type oxyde.

Selon une troisième variante de préparation dudit deuxième catalyseur zéolithique, chaque zéolithe est mise en forme indépendamment avec un liant. Le mélange des zéolithes peut être réalisé après mise en forme (extrudés ou grains). Les métaux sont déposés avant ou après mélange des zéolithes mises en forme, de préférence avant. Un métal différent peut ainsi être déposé sur les deux zéolithes mises en forme.

D'une manière générale, la préparation du premier et du deuxième catalyseurs zéolithiques se termine généralement par une calcination, préférentiellement sous flux d'air, dite calcination finale, habituellement à une température comprise entre 300 et 600 °C, de préférence précédée d'un séchage, par exemple à l'étuve, à une température généralement comprise entre la température ambiante et 250 °C, de préférence entre 40 et 200 °C. Ladite étape de séchage est de préférence menée pendant la montée en température nécessaire pour effectuer ladite calcination. On peut procéder ensuite à une réduction sous hydrogène, généralement à une température comprise entre 300 et 600°C, de préférence entre 350 et 550°C, et pour une durée comprise entre 1 et 10 heure(s), de préférence entre 2 et 5 heures. Une telle réduction peut avoir lieu *ex situ* ou *in situ,* par rapport au lieu d'utilisation desdits premier et deuxième catalyseurs zéolithiques.

Ledit premier et/ou deuxième catalyseur(s) zéolithique(s) utilisé(s) pour la mise en oeuvre respectivement des étapes a) et b) du procédé selon l'invention peu(ven)t éventuellement contenir du soufre. Dans ce cas, le soufre est introduit sur le(s) catalyseur(s) zéolthique(s) concerné(s), mis en forme, calciné(s), contenant le ou les élément(s) cité(s) précédemment, soit *in situ* avant la réaction catalytique, soit *ex situ.* La sulfuration s'effectue en utilisant tout agent sulfurant bien connu de l'homme de métier, tel que par exemple le disulfure de diméthyle ou le sulfure d'hydrogène. La sulfuration éventuelle intervient après la réduction. Dans le cas d'une sulfuration *in situ,* la réduction, si le catalyseur n'a pas été préalablement réduit, intervient avant la sulfuration. Dans le cas d'une sulfuration *ex situ,* on effectue la réduction puis la sulfuration.

Les exemples suivants illustrent la présente invention sans pour autant en limiter la portée.

### Exemple 1 : Préparation des catalyseurs à base de zéolithes 10 MR, à base de zéolithes à au moins 12 MR et à base d'une zéolithe 10 MR et d'une zéolithe à au moins 12 MR.

Les zéolithes utilisées pour préparer les différents catalyseurs employés pour mettre en oeuvre le procédé selon l'invention sont présentées dans le tableau 1 avec leur composition (rapport atomique Si / Al mesuré par fluorescence X (FX)) et leur teneur résiduelle en sodium. Les cinq zéolithes concernées sont sous forme acide.

Les zéolithes Béta, Mordénite et ZSM-5 sont des zéolithes commerciales (Zeolyst).

La zéolithe NU-87 a été synthétisée d'après la demande de brevet européen EP-A-0.377.291 ou le brevet EP-B-0.378.916. La zéolithe de départ possède un rapport atomique Si/Al global égal à 17,2, une teneur pondérale en sodium égale à 1256 ppm. Cette zéolithe NU-87 subit, tout d'abord, une calcination dite sèche à 550°C sous flux d'air et d'azote durant 6 heures. Puis, le solide obtenu est soumis à un échange ionique dans une solution de NH₄NO₃ 10N, à environ 100°C pendant 4 heures. La zéolithe NU-87 est alors soumise à un traitement par une solution d'acide nitrique 7N, à environ 100°C, pendant 5 heures. Le volume V de la solution d'acide nitrique engagé (en ml) est égal à 10 fois le poids P de zéolithe NU-87 sèche (V/P=10). Ce traitement par une solution d'acide nitrique 7N est réalisé une seconde fois dans les mêmes conditions opératoires. A l'issue de ces traitements, la zéolithe obtenue se trouve sous sa forme H et possède un rapport Si/Al atomique global égal à 33,3, et une teneur en Na de 10 ppm.

La zéolithe ITQ-23 est synthétisée selon la méthode décrite par A. Corma et al. in "Abstracts of 14th Int. Zeolite Conference", (Eds. E. van Stee, L. H. Callanan and M. Claeys), 25-30 April 2004, Cape Town, South Africa.

**Tableau 1: zéolithes 10 MR et zéolithes à au moins 12 MR**

| zéolithes | Si/Al (FX) | Na (ppm) | Type |
|---|---|---|---|
| Béta | 12,5 | 87 | 12 MR |
| ZSM-5 | 17,5 | 132 | 10 MR |
| mordénite | 10 | 109 | 12 MR |
| ITQ-23 | 20 | 19 | 10 & 12MR |
| NU-87 | 33,3 | 10 | 10 & 12MR |

Les zéolithes sont ensuite mises en forme par extrusion avec un gel d'alumine de manière à obtenir, après séchage à 120°C pendant une nuit et calcination sous air sec à 500°C pendant 4 heures, le support qui contient en poids 80 % de zéolithe et 20 % d'alumine. La partie zéolithique est constituée d'une zéolithe seule (10 MR ou 12 MR) ou d'un mélange mécanique de deux zéolithes différentes, réalisé avant la mise en forme.

Le support zéolithique comprenant une zéolithe ou un mélange de deux zéolithes différentes est soumis à une imprégnation à sec par une solution de précurseur métallique (perrhénate d'ammonium pour le rhénium, heptamolybdate d'ammonium pour le molybdène, nitrate de gallium pour le gallium) de manière à déposer un pourcentage de métal visé. Le solide humide est ensuite séché à 120°C pendant 12 heures et calciné sous un débit d'air sec à la température de 500°C pendant une heure. La composition des catalyseurs obtenus est reportée dans le tableau 2.

**Tableau 2 : catalyseurs contenant soit une zéolithe 10 MR soit une zéolithe à au moins 12 MR soit une zéolithe 10 MR et une zéolithe à au moins 12 MR**

| Catalyseur | %Al2O3 | zéolithe(s) | Zéolithes ratio | % Métal |
|---|---|---|---|---|
| A | 20 | ZSM-5 | 100 | 0,3% Re |
| B | 20 | mordénite | 100 | 0,3% Mo |
| C | 20 | NU-87 | 100 | 0,3% Re |
| D | 20 | béta | 100 | 0,3% Re |
| E | 20 | béta + ZSM-5 | 75/25 | 0,5% Re |
| F | 20 | NU-87 + ZSM-5 | 75/25 | 0,25% Re |
| G | 20 | ITQ-23+ ZSM-5 | 75/25 | 0,25% Ga |

### Exemple 2 : Performances catalytiques de catalyseurs zéolithiques utilisés pour réaliser la transalkylation d'une charge AC9+ dans un seul réacteur catalytique équipé d'une seule zone réactionnelle (comparatif).

On prépare un catalyseur de masse m1 en réalisant un mélange mécanique constitué de 50% poids du catalyseur A et 50% poids d'un second catalyseur (catalyseur D ou catalyseur C). Les catalyseurs A, C et D ainsi que les catalyseurs résultant du mélange de A et C et du mélange de A et D sont préalablement réduits sous hydrogène à 450°C pendant 2h.

On introduit le catalyseur résultant du mélange mécanique du catalyseur A et du catalyseur C dans un réacteur catalytique équipé d'une seule zone réactionnelle. On introduit à l'entrée du réacteur catalytique une charge constituée de 50% de toluène, 16% d'éthyltoluène, 28% de triméthylbenzène et 6% d'aromatiques à au moins 10 atomes de carbone.

Les tests catalytiques sont réalisés dans les conditions opératoires suivantes:
- température : 400°C
- pression totale : 25 bar
- H₂/HC = 8,5 mol/mol (HC désigne l'ensemble des hydrocarbures aromatiques initialement introduits)
- WHSV = 4h⁻¹ (masse de charge par masse de catalyseur et par heure)

On reproduit ce test en utilisant comme catalyseur dans la zone réactionnelle un catalyseur résultant du mélange mécanique du catalyseur A et du catalyseur D. Les résultats sont donnés dans le tableau ci-dessous.

| exemple | 2.1 | 2.2 |
|---|---|---|
| catalyseur | 50% A + 50% D | 50% A + 50% C |
| conversion globale (%) | 56,1 | 52,3 |
| Rendement en xylènes (%) | 33,0 | 26,3 |

### Exemple 3 : Performances catalytiques de catalyseurs zéolithiques utilisés pour réaliser la transalkylation d'une charge AC9+ dans deux réacteurs catalytiques équipés chacun d'une zone réactionnelle (conforme à l'invention).

On introduit un premier catalyseur zéolithique de masse m'1 dans le premier réacteur catalytique et un deuxième catalyseur zéolithique de masse m'2 dans le deuxième réacteur catalytique, placé en aval et en série du premier réacteur. Les proportions de m'1 par rapport à m1 et celles de m'2 par rapport à m1 sont données dans le tableau ci-dessous, m1 étant la masse du catalyseur utilisé dans l'exemple 2. Pour deux des tests réalisés dans cet exemple 3, m'1 + m'2 = m1. Pour le troisième test, m'1 + m'2 < m1.

On introduit à l'entrée du premier réacteur catalytique une charge aromatique constituée d'hydrocarbures AC9+ constituée de 32% d'éthyltoluène, 56% de triméthylbenzène et 12% d'aromatiques à au moins 10 atomes de carbone. On introduit, à l'entrée du deuxième réacteur catalytique, du toluène (même débit que la charge d'AC9+ injectée en entrée du premier réacteur catalytique) en mélange avec l'effluent issu du premier réacteur catalytique.

Les tests catalytiques sont réalisés dans les conditions opératoires suivantes :
- température : 400°C dans chacun des réacteurs
- pression totale : 25 bar dans chacun des réacteurs
- H₂/HC aromatiques = 8,5 mol/mol global
- La WHSV est donnée par la formule = (débit massique du toluène + débit massique d'AC9+) / (m'1 + m'2)

| exemple | 3.1 | 3.2 | 3.3 |
|---|---|---|---|
| 1^{er} catalyseur zéolithique | A | A | A |
| 2^{ème} catalyseur zéolithique | E | F | E |
| ratio m'l/ml | m'1 = 1/3 m1 | m'1 = 1/3 m1 | m'1 = 1/4 m1 |
| ratio m'2/m1 | m'2 = 2/3 m1 | m'2 = 2/3 m1 | m'2 = 1/2 m1 |
| conversion globale (%) | 52,2 | 49,3 | 51,9 |
| Rendement en xylènes (%) | 34,4 | 32,7 | 33,1 |
| WHSV (h⁻¹) | 4 | 4 | 5,3 |

La comparaison des exemples 2.1 et 3.1 montre que pour une même composition catalytique (zéolithes ZSM-5 et béta), une même quantité (m'1+m'2=m1) et une même proportion de zéolithes (50% poids de zéolithe ZSM-5 et 50% poids de zéolithe béta), le rendement en xylènes est sensiblement augmenté lorsque le toluène est introduit, non pas en mélange avec la charge AC9+ dans une seule zone réactionnelle, mais à l'entrée d'une deuxième zone réactionnelle placée en aval d'une première zone réactionnelle à l'entrée de laquelle est introduite la charge AC9+. Cet effet avantageux sur le rendement en xylènes est également observé en comparant les exemples 2.2 et 3.2. Par ailleurs, la comparaison des exemples 2.1 et 3.3 montre que le rendement en xylènes est maintenu lorsque deux zones réactionnelles sont utilisées conformément à l'invention alors que la quantité totale de zéolithes est plus faible (m'1+m'2<m1) ce qui permet ainsi de diminuer la quantité de catalyseurs zéolithiques nécessaire lorsqu'une performance déterminée est recherchée.

### Exemple 4 : Performances catalytiques de catalyseurs zéolithiques utilisés pour réaliser la transalkylation d'une charge AC9+ dans deux réacteurs catalytiques équipés chacun d'une zone réactionnelle (conforme à l'invention).

On introduit un premier catalyseur zéolithique de masse m"1 dans le premier réacteur catalytique et un deuxième catalyseur zéolithique de masse m"2 dans le deuxième réacteur catalytique, placé en aval et en série du premier réacteur.

On introduit à l'entrée du premier réacteur catalytique une charge aromatique constituée d'hydrocarbures AC9+ constituée de 32% d'éthyltoluène, 56% de triméthylbenzène et 12% d'aromatiques à au moins 10 atomes de carbone. On introduit, à l'entrée du deuxième réacteur catalytique, du toluène (même débit que la charge d'AC9+ injectée en entrée du premier réacteur catalytique) en mélange avec l'effluent issu du premier réacteur catalytique.

Les tests catalytiques sont réalisés dans les conditions opératoires suivantes :
- température : 400°C dans chacun des réacteurs
- pression totale : 25 bar dans chacun des réacteurs
- H₂/HC aromatiques = 8,5 mol/mol global
- La WHSV est donnée par la formule = (débit massique du toluène + débit massique d'AC9+) / (m"1 + m"2)

| exemple | 4.1 | 4.2 | 4.3 |
|---|---|---|---|
| 1^{er} catalyseur zéolithique | A | A | A |
| 2^{ème} catalyseur zéolithique | B | G | C |
| conversion globale (%) | 54,8 | 52,9 | 53,1 |
| Rendement en xylènes (%) | 31,8 | 32,4 | 29,5 |
| WHSV (h⁻¹) | 4 | 4 | 4 |

## Revendications

1. Procédé de production de xylènes par transalkylation d'une charge d'hydrocarbures alkylaromatiques ayant au moins 9 atomes de carbone par molécule comprenant :
a) l'introduction de ladite charge d'hydrocarbures alkylaromatiques à l'entrée d'une première zone réactionnelle où elle est mise en contact avec au moins un premier catalyseur zéolithique, les hydrocarbures alkylaromatiques ayant au moins 9 atomes de carbone par molécule représentant au moins 95% en volume de ladite charge,
b) l'introduction d'au moins une partie de l'effluent issu de l'étape a) et d'une charge contenant uniquement du toluène pur à l'entrée d'une deuxième zone réactionnelle contenant au moins un deuxième catalyseur zéolithique et
c) la séparation d'au moins une partie de l'effluent issu de l'étape b).

2. Procédé selon la revendication 1 dans lequel les conditions opératoires dans ladite première zone réactionnelle et ladite deuxième zone réactionnelle sont telles que la température y est comprise entre 250 et 650°C, la pression y est comprise entre 1 et 6 MPa, la vitesse spatiale d'alimentation y est comprise entre 0,05 et 20 h⁻¹ et le rapport molaire H₂/hydrocarbures aromatiques y est compris entre 1 et 30.

3. Procédé selon la revendication 1 ou 2 dans lequel ledit premier catalyseur zéolithique et ledit deuxième catalyseur zéolithique comprennent au moins une zéolithe choisie dans le groupe constituée par les zéolithes de type structural MOR, BEA, MFI, EUO, FAU, BOG, TON et NES.

4. Procédé selon la revendication 1 ou 2 dans lequel ledit premier catalyseur zéolithique et/ou ledit deuxième catalyseur zéolithique comprennent au moins une zéolithe IM-5.

5. Procédé selon l'une des revendications 1 à 4 dans lequel ledit premier catalyseur zéolithique et ledit deuxième catalyseur zéolithique comprennent une même et une seule zéolithe.

6. Procédé selon la revendication 5 dans lequel ladite zéolithe est une zéolithe de type structural MFI.

7. Procédé selon l'une des revendications 1 à 4 dans lequel ledit premier catalyseur zéolithique diffère dudit deuxième catalyseur zéolithique.

8. Procédé selon la revendication 7 dans lequel ledit premier catalyseur zéolithique comprend au moins une zéolithe présentant des canaux dont l'ouverture est définie par un anneau à 10 atomes d'oxygène et ledit deuxième catalyseur zéolithique comprend au moins une zéolithe présentant des canaux dont l'ouverture est définie par un anneau à 10 atomes d'oxygène et au moins une zéolithe présentant au moins des canaux ou des poches latérales dont l'ouverture est définie par un anneau à 12 atomes d'oxygène.

9. Procédé selon la revendication 8 dans lequel la zéolithe présentant des canaux dont l'ouverture est définie par un anneau à 10 atomes d'oxygène est choisie parmi les zéolithes ZSM-5, IM-5 et ZSM-22 et la zéolithe présentant au moins des canaux ou des poches latérales dont l'ouverture est définie par un anneau à 12 atomes d'oxygène est choisie parmi les zéolithes béta, Y, mordénite, NU-87, ITQ-23, EU-1 et la boggsite.

10. Procédé selon l'une des revendications 1 à 9 dans lequel ledit premier catalyseur zéolithique et/ou ledit deuxième catalyseur zéolithique comprend / comprennent au moins un métal choisi dans le groupe constitué par les métaux des groupes IIIA, VIB, VIIB et VIII.

11. Procédé selon la revendication 10 dans lequel le métal est le rhénium.

12. Procédé selon l'une des revendications 1 à 9 dans lequel on utilise dans l'étape a) un premier catalyseur zéolithique comprenant au moins un premier métal choisi dans le groupe constitué par les métaux des groupes IIIA, VIB, VIIB et VIII et dans l'étape b) un deuxième catalyseur zéolithique comprenant au moins un deuxième métal, différent dudit premier métal, et choisi dans le groupe constitué par les métaux des groupes IIIA, VIB, VIIB et VIII.

13. Procédé selon l'une des revendications 1 à 12 dans lequel ledit premier catalyseur zéolithique et/ou ledit deuxième catalyseur zéolithique contient du soufre.

## Claims

1. A process for producing xylenes by transalkylation of an alkyl-aromatic hydrocarbon feedstock having at least 9 carbon atoms per molecule comprises: a) introducing said alkyl-aromatic hydrocarbon feedstock at the inlet of a first reaction zone where it is brought into contact with at least a first zeolitic catalyst, alkyl-aromatic hydrocarbons having at least 9 carbon atoms per molecule representing at least 95% by volume of the feedstock, b) introducing at least a portion of resultant effluent obtained from stage a) and a feedstock that only contains pure toluene to the inlet of a second reaction zone that contains at least a second zeolitic catalyst, and c) separating at least a portion of resultant effluent obtained from stage b).

2. A process according to claim 1 conducted under operating conditions in said first reaction zone and said second reaction zone such that the temperature is between 250 and 650 degrees C., the pressure is between 1 and 6 MPa, the feed volumetric flow rate is between 0.05 and 20 h -1, and the H 2/aromatic hydrocarbon molar ratio is between 1 and 30.

3. A process according to claim 1 or claim 2, in which said first zeolitic catalyst and said second zeolitic catalyst comprise at least one zeolite that is selected from the group that consists of the zeolites of the MOR, BEA, MFI, EUO, FAU, BOG, TON and NES structural type.

4. A process according to claim 1 or claim 2, in which said first zeolitic catalyst and/or said second zeolitic catalyst comprise at least one IM-5 zeolite.

5. A process according to one of claims 1 to 4, in which said first zeolitic catalyst and said second zeolitic catalyst comprise one and the same zeolite.

6. A process according to claim 5, in which said zeolite is an MFI-structural-type zeolite.

7. A process according to one of claims 1 to 4, in which said first zeolitic catalyst differs from said second zeolitic catalyst.

8. A process according to claim 7, in which said first zeolitic catalyst comprises at least one zeolite having channel openings defined by a ring with 10 oxygen atoms, and said second zeolitic catalyst comprises at least one zeolite having channel openings defined by a ring with 10 oxygen atoms and at least one zeolite having at least channels or lateral pockets whose openings are defined by a ring with 12 oxygen atoms.

9. A process according to claim 8, in which the zeolite that has channels whose openings are defined by a ring with 10 oxygen atoms is selected from the ZSM-5, IM-5 and ZSM-22 zeolites, and the zeolite that has at least channels or lateral pockets whose openings are defined by a ring with 12 oxygen atoms is selected from the beta, Y, mordenite, NU-87, ITQ-23, and EU-1 zeolites and boggsite.

10. A process according to one of claims 1 to 9, in which said first zeolitic catalyst and/or said second zeolitic catalyst comprises/comprise at least one metal that is selected from the group that consists of the metals of groups IIIA, VIB, VIIB and VIII.

11. A process according to claim 10, in which the metal is rhenium.

12. A process according to one of claims 1 to 9, in which in stage a), said first zeolitic catalyst comprises at least a first metal selected from the group that consists of the metals of groups IIIA, VIB, VIIB and VIII, and in stage b), a said second zeolitic catalyst comprising at least a second metal, different from said first metal, and is selected from the group that consists of the metals of groups IIIA, VIB, VIIB and VIII, is used.

13. A process according to one of claims 1 to 12, in which said first zeolitic catalyst and/or said second zeolitic catalyst contain(s) sulfur.

## Patentansprüche

1. Verfahren zur Produktion von Xylenen durch Transalkylierung einer Beschickung aus alkylaromatischen Kohlenwasserstoffen, die mindestens 9 Kohlenstoffatome je Molekül haben, umfassend:
a) Einführen der Beschickung aus alkylaromatischen Kohlenwasserstoffen in den Einlass einer ersten Reaktionszone, in der sie mit mindestens einem ersten zeolithischen Katalysator in Kontakt gebracht wird, wobei die alkylaromatischen Kohlenwasserstoffe, die mindestens 9 Kohlenstoffatome je Molekül haben, mindestens 95 Vol.-% der Beschickung darstellen,
b) Einführen mindestens eines Teils des aus Schritt a) stammenden Abflusses und einer Beschickung, die ausschließlich reines Toluen enthält, in den Einlass einer zweiten Reaktionszone, die mindestens einen zweiten zeolithischen Katalysator enthält, und
c) Abscheiden mindestens eines Teils des aus Schritt b) stammenden Abflusses.

2. Verfahren nach Anspruch 1, wobei die Betriebsbedingungen in der ersten Reaktionszone und der zweiten Reaktionszone so sind, dass die Temperatur dort im Bereich zwischen 250 und 650 °C, der Druck dort im Bereich zwischen 1 und 6 MPa, die Versorgungsraumgeschwindigkeit dort im Bereich zwischen 0,05 und 20 h⁻¹ und das Molverhältnis von H₂ zu aromatischen Kohlenwasserstoffen dort im Bereich zwischen 1 und 30 liegt.

3. Verfahren nach Anspruch 1 oder 2, wobei der erste zeolithische Katalysator und der zweite zeolithische Katalysator mindestens einen Zeolithen umfassen, der aus der Gruppe gebildet aus den Zeolithen vom Strukturtyp MOR, BEA, MFI, EUO, FAU, BOG, TON und NES ausgewählt ist.

4. Verfahren nach Anspruch 1 oder 2, wobei der erste zeolithische Katalysator und/oder der zweite zeolithische Katalysator mindestens einen IM-5-Zeolithen umfassen.

5. Verfahren nach einem der Ansprüche 1 bis 4, wobei der erste zeolithische Katalysator und der zweite zeolithische Katalysator einen gleichen und einzigen Zeolithen umfassen.

6. Verfahren nach Anspruch 5, wobei der Zeolith ein Zeolith vom Strukturtyp MFI ist.

7. Verfahren nach einem der Ansprüche 1 bis 4, wobei der erste zeolithische Katalysator sich von dem zweiten zeolithischen Katalysator unterscheidet.

8. Verfahren nach Anspruch 7, wobei der erste zeolithische Katalysator mindestens einen Zeolithen umfasst, der Kanäle aufweist, deren Öffnung durch einen Ring mit 10 Sauerstoffatomen definiert ist, und der zweite zeolithische Katalysator mindestens einen Zeolithen, der Kanäle aufweist, deren Öffnung durch einen Ring mit 10 Sauerstoffatomen definiert ist, und mindestens einen Zeolithen umfasst, der mindestens Kanäle oder seitliche Taschen aufweist, deren Öffnung durch einen Ring mit 12 Sauerstoffatomen definiert ist.

9. Verfahren nach Anspruch 8, wobei der Zeolith, der Kanäle aufweist, deren Öffnung durch einen Ring mit 10 Sauerstoffatomen definiert ist, aus den Zeolithen ZSM-5, IM-5 und ZSM-22 ausgewählt ist, und der Zeolith, der mindestens Kanäle oder seitliche Taschen aufweist, deren Öffnung durch einen Ring mit 12 Sauerstoffatomen definiert ist, aus den Zeolithen Beta, Y, Mordenit, NU-87, ITQ-23, EU-1 und Boggsit ausgewählt ist.

10. Verfahren nach einem der Ansprüche 1 bis 9, wobei der erste zeolithische Katalysator und/oder der zweite zeolithische Katalysator mindestens ein Metall, ausgewählt aus der Gruppe bestehend aus den Metallen der Gruppen IIIA, VIB, VIIB und VIII umfasst / umfassen.

11. Verfahren nach Anspruch 10, wobei das Metall Rhenium ist.

12. Verfahren nach einem der Ansprüche 1 bis 9, wobei in Schritt a) ein erster zeolithischer Katalysator, der mindestens ein erstes Metall, ausgewählt aus der Gruppe bestehend aus den Metallen der Gruppen IIIA, VIB, VIIB und VIII umfasst, und in Schritt b) ein zweiter zeolithischer Katalysator verwendet wird, der mindestens ein zweites Metall umfasst, das sich von dem des ersten Metalls unterscheidet, und aus der Gruppe bestehend aus den Metallen der Gruppen IIIA, VIB, VIIB und VIII ausgewählt ist.

13. Verfahren nach einem der Ansprüche 1 bis 12, wobei der erste zeolithische Katalysator und/oder der zweite zeolithische Katalysator Schwefel enthält.
